Europäisches Patentamt

European Patent Office

Office européen des brevets

(11) Veröffentlichungsnummer: **0 407 698 A1**

# EUROPÄISCHE PATENTANMELDUNG

(21) Anmeldenummer: 90108472.3

(22) Anmeldetag: 05.05.90

(51) Int. Cl.⁵: **A61L 27/00**

(30) Priorität: 08.05.89 DE 3914999

(43) Veröffentlichungstag der Anmeldung:
16.01.91 Patentblatt 91/03

(84) Benannte Vertragsstaaten:
BE CH DE FR GB LI LU NL

(71) Anmelder: BATTELLE-INSTITUT E.V.
Am Römerhof 35 Postfach 90 01 60
D-6000 Frankfurt am Main 90(DE)

(72) Erfinder: Heide, Helmut, Dr.
Am Hohenstein 14
D-6233 Kelkheim-Fischbach(DE)
Erfinder: Schwämmlein, Wolfgang
Kardinal von Galen Strasse 26
D-6500 Mainz 32(DE)

(54) Verfahren zur Herstellung eines implantierbaren Knochenersatz-werkstoffs auf der Basis von Hydroxylapatit.

(57) Beschrieben wird ein Verfahren zur Herstellung eines implantierbaren Knochenersatzwerkstoffs, wobei Hydroxylapatit direkt auf einen metallischen Träger durch Plasmaspritzen aufgebracht wird. Damit ist der Vorteil verbunden, daß auch komplizierte Geometrien mit dem Hydroxylapatit beschichtet werden können. Nachteilig am Plasmaspritzverfahren ist allerdings die hohe Restporosität des Hydroxylapatits, dessen geringe Haftung auf dem metallischen Träger und seine undefinierte kristallchemische Zusammensetzung. Um diese Nachteile zu beseitigen, wird das aufgespritzte Hydroxylapatit daher anschließend hydrothermal nachbehandelt. Dies führt zu einer Rehydration und kristallchemischen Konsolidierung des undefinierten Plasma-Hydroxylapatits. In einem dritten Verfahrensschritt wird die Hydroxylapatitoberfläche schließlich durch heißisostatisches Pressen bis zur theoretischen Dichte gebracht und über eine diffusivere Bindung fest mit dem Trägermaterial verbunden.

EP 0 407 698 A1

## VERFAHREN ZUR HERSTELLUNG EINES IMPLANTIERBAREN KNOCHENERSATZWERKSTOFFS AUF DER BASIS VON HYDROXYLAPATIT

Die Erfindung betrifft ein Verfahren zur Herstellung eines implantierbaren Knochenersatzwerkstoffs, wobei auf einen metallischen Träger eine Schicht aus Hydroxylapatit aufgebracht wird, die anschließend durch heißisostatisches Pressen verdichtet und mit dem Träger fest verbunden wird.

Ein derartiges Verfahren beschreibt die DE-OS 34 47 583. Dieses bekannte Verfahren hat den Vorteil, daß durch das heißisostatische Pressen das hocherwünschte Hydroxylapatit praktisch bis zu seiner theoretischen Dichte gebracht und über eine diffusive Bindung fest mit dem Substrat verbunden werden kann, und zwar ohne einen auch sonst beim Stand der Technik verwendeten Haftvermittler, der sich aber gewebefeindlich verhält. Nachteilig bei dem in dieser DE-OS beschriebenen Verfahren ist es allerdings, daß nur Substrate mit einfachen Geometrien entsprechend beschichtet werden können.

Komplizierte Geometrien lassen sich nach dem Stand der Technik mit Hilfe des Plasmaspritzverfahrens mit Hydroxylapatit (HA) beschichten. Hierzu benötigt man bisher aber einen Haftvermittler, der sich gewebefeindlich verhält, oder man erreicht keine ausreichende Haftung der Beschichtung auf dem Trägermaterial. Dieser Stand der Technik ist beispielsweise geschildert im ersten Absatz der Seite 5 der erwähnten DE-OS.

Weiterhin ist beim Plasmaspritzen nachteilig die zumindest partielle thermische Zersetzung des OH-haltigen HA, die beim Plasmaspritzen zur Ausbildung unstöchiometrischer Phasen führt. Außerdem sind Plasmaspritzschichten generell porös; sie sind nur unbefriedigend stabil und haften nur mechanisch am Substrat. Das Plasmaspritzen allein bietet also keine werkstofftechnisch befriedigende Lösung.

Das Plasmaspritzen ist heute die gängige Technik zur Beschichtung von Prothesenmetallen und wird z.B. in einer Übersichtsarbeit von E.Dörre (Biomed. Technik, 34 (1989) 46-52) beschrieben.

Die Umwandlung von wasserfreien Kalziumphosphaten, wie z.B. $\alpha$- und $\beta$-Trikalziumphosphat in Hydroxylapatit mittels heißem Wasser, wird z. B. in der DE 37 11 426 beschrieben.

In der DE 34 47 583 C2 wird die Nachverdichtung von Hydroxylapatit durch heißisostatisches Pressen (HIP) behandelt.

Während diese Druckschrift bereits das HIP-Verfahren als Beschichtungstechnik nutzt, beschreibt die GB 21 30 187 den Vorteil der Anwendung von Druck und Temperatur zur Erzielung dichter HA-Qualitäten, hier allerdings als Verbundwerkstoff mit Faserverstärkung.

Der Erfindung liegt daher die Aufgabe zugrunde, ein Verfahren zur Herstellung eines implantierbaren Knochenersatzwerkstoffes vorzuschlagen, bei dem ein mechanisch entsprechend belastbarer metallischer Träger mit dem biologisch hoch erwünschten Hydroxylapatit beschichtet wird, wobei auch komplizierte Geometrien dauerhaft beschichtet werden können und man ohne einen Haftvermittler auskommt. Die Beschichtung soll allen Anforderungen der Praxis an eine Langzeit-Prothese entsprechen und insbesondere mechanisch hochbelastbar sein. Die bekannten Eigenschaften von Hydroxylapatit, nämlich Biokompatibilität, Bioaktivität, chemische, biologische und physiologische Stabilität von Hydroxylapatit sollen ungeschmälert beibehalten bleiben.

Ausgehend von einem Verfahren mit den eingangs genannten Merkmalen gelingt dies gemäß der Erfindung dadurch, daß das Hydroxylapatit durch Plasmaspritzen direkt auf den Träger aufgebracht und anschliessend zu seiner Rehydration und kristallchemischen Konsolidierung hydrothermal nachbehandelt wird, worauf das heißisostatische Pressen erfolgt.

Durch das erfindungsgemäß durchgeführte Plasmaspritzen lassen sich selbst komplexe Geometrien problemlos beschichten. Die hierbei an und für sich nachteilige hohe Restporosität des aufgespritzten Hydroxylapatits, seine geringe Haftung und die undefinierte kristallchemische Zusammensetzung des plasmaaufgespritzten Hydroxylapatits werden durch die nachfolgenden Verfahrensschritte beseitigt. Die hydrothermale Nachbehandlung bewirkt nämlich die Rehydration und kristallchemische Konsolidierung des undefinierten Plasma-Hydroxylapatits. Das anschließende, heißisostatische Pressen bringt die Oberfläche des Hydroxylapatit bis etwa zur theoretischen Dichte dieses Materials und die Beschichtung wird über eine diffusive Bindung fest mit dem Substrat verbunden, so daß das Hydroxylapatit beim erfindungsgemäßen Verfahren auch ohne einen nachteiligen Haftvermittler fest und dauerhaft am Substrat haftet.

Die hydrothermale Nachbehandlung wird man in Gegenwart von Wasser bei Temperaturen und Drücken durchführen, die den erwähnten Zweck erfüllen. Versuche haben ergeben, daß sich hierbei Temperaturen und Drücke in den in Patentanspruch 2 bzw. 3 angegebenen Bereichen besonders gut eignen.

Die Erfindung wird im folgenden anhand eines Ausführungsbeispiels näher erlautert, aus dem sich weitere wichtige Merkmale ergeben.

Trikalziumphosphat wird bei 200°C in Gegenwart von Wasser in einem Autoklaven (Druck ca. 2 MPa) behandelt und somit in einen stöchiometrisch aufgebauten Hydroxylapatit (HA) überführt (CA2-(PO4)3(OH)). Hierbei entsteht ein mehr oder weniger gleichmäßig agglomeriertes Korn, aus dem eine Siebfraktion von 45 bis 124 μm abgesiebt wird, die als rieselfähige Fraktion für den Plasmaspritzprozess zunächst verwendet wird. Eventuell vorhandenes Überkorn wird weiter zerkleinert und ebenfalls wie oben abgesiebt und zu der Hauptmenge zurückgeführt.

**Das Plasmaspritzen**

Das Plasmaspritzen auf aufgerauhten metallischen Grundkörpern (Kobalt/Chrom/Basislegierung) erfolgt mit N2/H2 Atmosphäre bei Temperaturen von ca. 3000° C. Die Plasmaschicht erreicht durch mehrmaliges Überspritzen eine Schichtdicke von ca. 200 μm. Dieses Material besitzt eine Gefügeporosität von ca. 10 % und haftet auf der Unterlage nur mechanisch (Abzugsfestigkeit ca. 0,5 bis 1 MPa). Nach Ausweis der Röntgenanalyse besteht diese Schicht aus schlecht kristallisiertem HA, teilentwässerten Kalziumphosphaten, teilweise in Form von β TCP und glasiger, amorpher Phase. (Der Anteil an Fremdphasen kann bis zu 50 Volumenprozent betragen).

**Die hydrothermale Nachbehandlung**

Die so beschichteten Grundkörper werden einer hydrothermalen Reaktion unterworfen, die zuvor schon beim Ausgangsrohstoff beschrieben wurde. Hierbei setzt sich der durch den Plasmaprozess kristallchemisch gestörte, teilentwässerte "Plasma-HA" quantitativ in wohldefinierten HA um.

**Die HIP-Verdichtung**

Diese Beschichtung wird zur endgültigen HIP-Nachbehandlung in hexagonales Bornitrid eingehüllt (Reaktionsbarriere) und in einer Glasampulle eingeschmolzen. Diese Anordnung wird in einer Heißisostatpresse bei 850° C und 160 MPa nachverdichtet, indem die Glasampulle zunächst drucklos bis zur Erweichung bei 600° erwärmt und dann im weiteren Verlauf bis auf die o.a. Endbedingungen gebracht wird.

Bei diesem Vorgang wird der HA auf 100% der theoretischen Dichte gebracht, ohne daß es - wie bei der drucklosen Aufsinterung - zur thermischen Zersetzung des HA kommt. Außerdem ist der HA nach dieser Behandlung über eine chemische Bindung mit dem Substrat verbunden. Die Abzugsfestigkeit beträgt nunmehr 40 bis 50 MPa, was nach allgemeiner Erfahrung für die Beschichtung einer Prothese mehr als ausreichend ist.

Der nach diesem Verfahren hergestellte Knochenersatzwerkstoff zeichnet sich also durch ausreichende mechanische Eigenschaften aus (Abzugsfestigkeit, Biegefestigkeit, Rißzähigkeit, Ermüdungsverhalten usw.) Er besitzt die geforderte Gefügedichte zur Abschirmung der biologischen Sphäre von dem Metall-HA-Übergangsbereich. Er hat eine reproduzierbare und definierte chemische und kristallografische Beschaffenheit der HA-Grenzfläche zum Knochen und er zeichnet sich durch chemische Stabilität im biologischen Milieu aus. Die nach diesem Verfahren hergestellte Beschichtung besteht aus stöchiometrisch aufgebautem HA. In in-vitro und in-vivo-tests hat sich der Knochenersatzwerkstoff als optimal bioverträglich erwiesen.

**Ansprüche**

1. Verfahren zur Herstellung eines implantierbaren Knochenersatzwerkstoffs, wobei auf einen metallischen Träger eine Schicht aus Hydroxylapatit aufgebracht wird, die anschließend durch heißisostatisches Pressen verdichtet und mit dem Träger fest verbunden wird,
**dadurch gekennzeichnet,**
daß das Hydroxylapatit durch Plasmaspritzen direkt auf den. Träger aufgebracht und anschließend zu seiner Rehydration und kristallchemischen Konsolidierung hydrothermal nachbehandelt wird, worauf das heißisostatische Pressen erfolgt.
2. Verfahren nach Anspruch 1,
**dadurch gekennzeichnet,**
daß die hydrothermale Nachbehandlung im Temperaturbereich von 50 bis 250° C und bei Drücken von 0,5 bis 3 MPa in Gegenwart von Wasser erfolgt.
3. Verfahren nach Anspruch 2,
**dadurch gekennzeichnet,**
daß die hydrothermale Nachbehandlung bei 200° C und bei 2MPa erfolgt.

Europäisches Patentamt

# EUROPÄISCHER RECHERCHENBERICHT

Nummer der Anmeldung

EP 90 10 8472

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,Y | EP-A-0 285 826 (BENCKISER-KNAPSACK) * Ansprüche 1,2 * --- | 1-3 | A 61 L 27/00 |
| D,Y | WO-A-8 603 977 (BATTELLE-INSTITUT) * Anspruch 1 * --- | 1 | |
| Y | DE-A-3 709 457 (PERMETEC ELECTRODE) * Seite 5, Zeilen 25-31 * ----- | 2,3 | |

| | RECHERCHIERTE SACHGEBIETE (Int. Cl.5) |
|---|---|
| | A 61 L |

Der vorliegende Recherchenbericht wurde für alle Patentansprüche erstellt

| Recherchenort | Abschlußdatum der Recherche | Prüfer |
|---|---|---|
| DEN HAAG | 05-09-1990 | PELTRE CHR. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
..........................................................................................
& : Mitglied der gleichen Patentfamilie, übereinstimmendes Dokument

EPO FORM 1503 03.82 (P0403)